# DEMANDE DE BREVET EUROPEEN

(11) **EP 4 176 847 A1**
(43) Date de publication de la demande: **10.05.2023**
(21) Numéro de dépôt: 22205458.7
(22) Date de dépôt: 04.11.2022
(51) Int. Cl.: A61F 2/30, A61F 2/40

(54) **MÉTAGLÈNE POURVUE D'UN SUPPORT DE GREFFON PERFORÉ**

(30) Priorité: 04.11.2021 FR 2111694
(71) Demandeur: FX Solutions, 01000 Bourg en Bresse (FR)
(72) Inventeur: DORDAIN, Franck, 14000 CAEN (FR); ANTONI, Maxime, 68350 Brunstatt (FR); BARGOIN, Kevin, 44400 NANTES (FR); COLLIN, Philippe, 75006 Paris (FR); TORRENS, Carlos, 08860 Castelldefels (ES)
(74) Mandataire: LLR

(57) **Abrégé**

L'invention concerne une métaglène (3) d'un implant glénoïdien (1) de prothèse d'épaule inversée, destinée à être assemblée avec une glénosphère (2), la métaglène (3) comprenant une base (4) de forme cylindrique, un plot (6) de forme cylindrique et un support de greffon (5) de forme cylindrique présentant au moins un orifice traversant (7), la base (4), le plot (6) et le support de greffon (5) ayant un axe de révolution (A) commun.

## Description

L'invention concerne un implant d'essai glénoïdien de prothèse totale de l'épaule inversée, destiné à être fixé temporairement à la cavité glénoïdienne du patient.

Une prothèse totale de l'épaule inversée comprend un implant huméral, c'est-à-dire une partie fixée dans l'humérus du patient, et un implant glénoïdien, c'est-à-dire une partie fixée sur la cavité glénoïdienne (aussi appelée glène) du patient. Ces deux implants sont articulés l'un avec l'autre afin de restaurer la mobilité du bras du patient opérée.

Dans le cadre d'une prothèse totale de l'épaule dite « inversée », le centre de rotation de l'articulation est déplacé sur l'implant glénoïdien, à l'inverse d'une prothèse anatomique où il demeure situé sur l'implant huméral.

Cette modification du positionnement du centre de rotation de l'articulation engendre une modification des muscles sollicités pour mouvoir le bras. En l'espèce, c'est le muscle deltoïde qui est sollicité tandis que pour une prothèse anatomique, les muscles sollicités demeurent ceux formant la coiffe des rotateurs, à savoir les muscles : sous-scapulaire, sus-épineux, sous-épineux et petit rond en plus du deltoïde. Cette modification des muscles impliqués peut s'expliquer par un endommagement irréparable de tout ou partie des muscles de la coiffe des rotateurs, un tel endommagement pouvant intervenir chez les personnes âgées notamment.

La prothèse d'épaule inversée implique donc la sollicitation du muscle deltoïde qui est ancré sur l'humérus de manière plus distale que les muscles de la coiffe des rotateurs. Par conséquent, il est nécessaire pour que le muscle deltoïde soit fonctionnel dès les premiers degrés d'abduction de déplacer le centre de rotation de la prothèse sur la glène. De plus pour utiliser le deltoïde pour les rotations interne et externe, il est nécessaire de le tendre. Une telle tension du muscle deltoïde peut être réalisée en latéralisant et/ou allongeant longitudinalement l'humérus. En d'autres termes, on latéralise et/ou on abaisse l'humérus par rapport à sa position naturelle afin d'augmenter les tensions qui s'appliquent sur le muscle deltoïde.

Ce déport latéral et/ou longitudinal du centre de rotation ainsi située sur l'implant glénoïdien peut être obtenu par une augmentation de l'épaisseur totale de l'implant, notamment par une augmentation de l'épaisseur de la métaglène.

La glène sur laquelle doit être fixée la métaglène est un os très plat et très fin qui s'use dans le temps. Cette usure, qui rend la fixation de la métaglène d'autant plus difficile, peut être symétrique ou asymétrique et plus ou moins prononcée. Pour fixer la métaglène (dont la base est plate) sur la glène du patient, le chirurgien doit préalablement araser la glène, ce qui équivaut à supprimer un peu d'os généralement sur les pourtours de la glène, pour pouvoir y fixer la métaglène via des vis.

En fonction de la typologie d'usure de la glène, il est connu d'utiliser différents types de métaglène. Certaines de ces métaglènes sont qualifiées de « latéralisées » et comportent un élément supplémentaire d'épaisseur constante permettant de déporter le centre de rotation de l'articulation à l'emplacement souhaité. Lorsque l'usure de la glène est asymétrique, il est connu d'utiliser d'autres métaglènes de type « wedge » qui comportent un élément supplémentaire asymétrique dont la forme permettant également de déporter le centre de rotation de l'articulation à l'emplacement souhaité. Néanmoins, les métaglènes existantes sont généralement en métal et, bien qu'elles permettent de déporter le centre de rotation au bon emplacement, elles ne favorisent pas la repousse osseuse, ce qui peut engendrer, à court ou moyen terme, une mauvaise fixation de la métaglène sur la glène du patient et, par conséquent, un risque que la prothèse doive être changée rapidement.

Pour répondre à cette problématique, il est connu d'utiliser comme élément supplémentaire un greffon osseux qui permet de placer le centre de rotation au bon endroit, dans le bon axe et de permettre la reconstitution du stock osseux entre la glène et la métaglène. Néanmoins, ce greffon osseux représente un élément fragile qui peut être déformé, voire même écrasé, lors de l'impaction de la métaglène dans la glène. En conséquence et lorsque le greffon est déformé ou écrasé, le centre de rotation de l'articulation n'est pas placé au bon emplacement, la reconstitution du stock osseux ne se fait pas et le chirurgien est dans l'obligation d'utiliser une nouvelle métaglène. Une telle situation n'est pas acceptable.

L'invention a notamment pour but de remédier à l'ensemble des problématiques précités en fournissant une métaglène qui, à la fois, favorise le bon positionnement du centre de rotation de l'articulation, évite la déformation ou l'écrasement du greffon osseux et permet une bonne reconstitution du stock osseux. Cela permet *in fine* de proposer une métaglène robuste et durable dans le temps.

A cet effet l'invention a pour objet une métaglène d'un implant glénoïdien de prothèse d'épaule inversée, destinée à être assemblée avec une glénosphère, la métaglène comprenant :
- une base de forme cylindrique et configurée pour être insérée au sein d'un logement de la glénosphère,
- un plot de forme cylindrique s'étendant depuis le centre de la base selon un sens d'impaction de la métaglène dans la glène d'un patient,
- un support de greffon de forme cylindrique et s'étendant depuis le pourtour de la base selon le sens d'impaction de la métaglène, le support de greffon présentant au moins un orifice traversant, la base, le plot et le support de greffon ayant un axe de révolution commun.

Ainsi, la métaglène selon l'invention permet de garantir l'intégrité du greffon lors de l'impaction de la métaglène au sein de la glène du patient, permettant ainsi une bonne reconstitution osseuse au fil du temps, tout en garantissant également le bon positionnement du centre de rotation de l'articulation. En effet, le support de greffon offre une protection pour ce dernier et le préserve des contraintes issues de l'impaction de la métaglène dans la glène. En outre, la présence de l'orifice traversant au sein dudit support de greffon offre une bonne communication entre les cellules osseuses du greffon et les cellules osseuses de la glène du patient.

Afin d'optimiser la reconstitution du stock osseux, le support de greffon comprend une pluralité d'orifices traversants répartie sur la surface interne dudit support.

Afin de permettre de latéraliser le centre de rotation de l'articulation, le support de greffon s'étend radialement d'une longueur constante. Avantageusement, la longueur constante est comprise entre 2 et 12mm.

Afin de permettre au chirurgien de s'adapter à une usure asymétrique de la glène du patient à opérer, le support de greffon s'étend radialement d'une longueur qui varie localement. Avantageusement, la variation maximale entre deux extrémités du support de greffon est comprise entre 2 et 20mm.

Afin d'optimiser la fabrication de la métaglène et ainsi réduire les coûts afférents, la base, le plot et le support de greffon sont monoblocs, de préférence réalisés en alliage de titane, de préférence encore réalisés en alliage de titane revêtu de titane et/ou de HAP.

Afin de permettre une meilleure fixation de la métaglène dans la glène du patient, le plot comprend, à son extrémité distale, un élément rapporté permettant d'allonger le plot. Alternativement, le plot comprend une vis dont l'extrémité s'étend au-delà de l'extrémité distale du plot permettant d'allonger le plot.

L'invention a également pour objet un procédé de fixation d'un implant glénoïdien de prothèse d'épaule inversée comprenant une métaglène selon l'une quelconque des revendications précédentes, comprenant les étapes suivantes :
- arasage de la glène ;
- impaction de la métaglène dans la glène ;
- mise en place des vis de fixation ; et
- impaction et vissage d'une glénosphère sur la métaglène.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue en perspective (figure 1A), une vue éclatée en perspective (figure 1B) et une vue éclatée de coté (figure 1C) représentant un implant glénoïdien de prothèse d'épaule inversée comprenant une métaglène selon un premier mode de réalisation ;
[Fig. 2] la figure 2 est un ensemble de vues de coté (figure 2A et 2B) représentant deux variantes de la métaglène de la figure 1 ;
[Fig. 3] la figure 3 est une vue en perspective (figure 3A), une vue éclatée en perspective (figure 3B) et une vue éclatée de coté (figure 3C) représentant un implant glénoïdien de prothèse d'épaule inversée comprenant une métaglène selon un deuxième mode de réalisation
[Fig. 4] la figure 4 est un ensemble de vues de coté (figure 4A, 4B et 4C) représentant deux variantes de la métaglène de la figure 3.

### Description détaillée

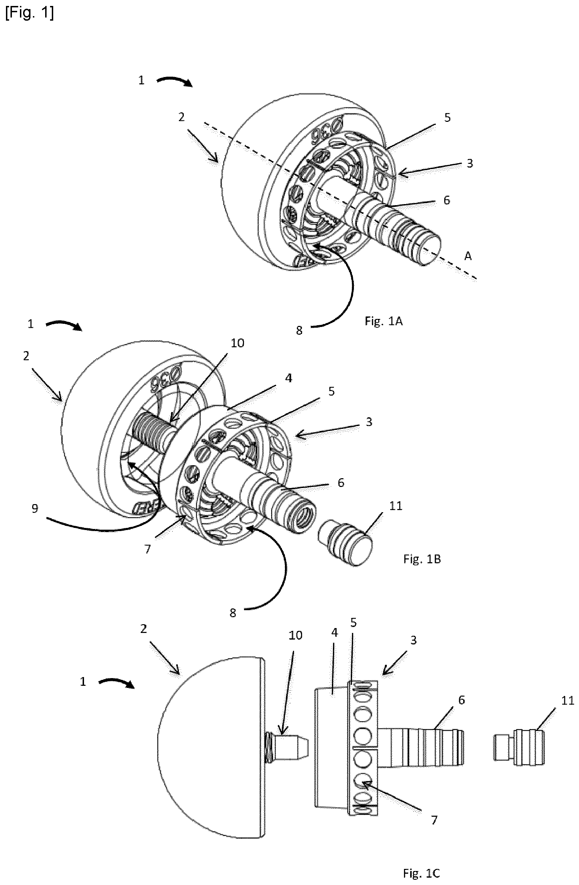

On a représenté sur les figures 1A à 4C une métaglène selon différents modes et variantes de réalisation de l'invention, la métaglène étant désignée par la référence générale 3.

Pour les deux modes de réalisation illustrés, ainsi que pour ceux non illustrés, la métaglène 3 d'un implant glénoïdien 1 de prothèse d'épaule inversée est destinée à être assemblée avec une glénosphère 2 et comprend :
- une base 4 de forme cylindrique et configurée pour être insérée au sein d'un logement 9 de la glénosphère 2 ;
- un plot 6 de forme cylindrique s'étendant depuis le centre de la base 4 selon un sens d'impaction de la métaglène 3 dans la glène d'un patient (non représentée) ;
- un support de greffon 5 de forme cylindrique et s'étendant depuis le pourtour de la base 4 selon le sens d'impaction de la métaglène 3, le support de greffon 5 présentant au moins un orifice traversant 7.
De plus, la base 4, le plot 6 et le support de greffon 5 ont un axe de révolution A commun.

La métaglène 3 selon l'invention est destinée à être assemblée avec une glénosphère 2 afin de former un implant glénoïdien 1 qui sera quant à lui en interaction avec un implant humérale (non représenté) pour former la prothèse d'épaule inversée.

L'assemblage entre la métaglène 3 et la glénosphère 2 est réalisé par l'intermédiaire de la vis de serrage 10 dont l'extrémité distale vient se visser dans l'extrémité proximale du plot 6. Afin d'assurer un assemblage encore meilleur, un cône morse (non représenté) est prévu entre la glénosphère 2 et la métaglène 3. La glénosphère 2 comprend un logement 9 dimensionné pour recevoir la base 4 de la métaglène 2. La métaglène 3 est en outre destinée à être impactée dans la glène du patient à opérer, après que le chirurgien ait arasé une ou plusieurs portions de cette dernière. Plus précisément, le plot 6 de la métaglène 3 pénètre dans la glène du patient lors de l'impaction de la métaglène 2 et on visse cette dernière sur la glène via des vis de fixation.

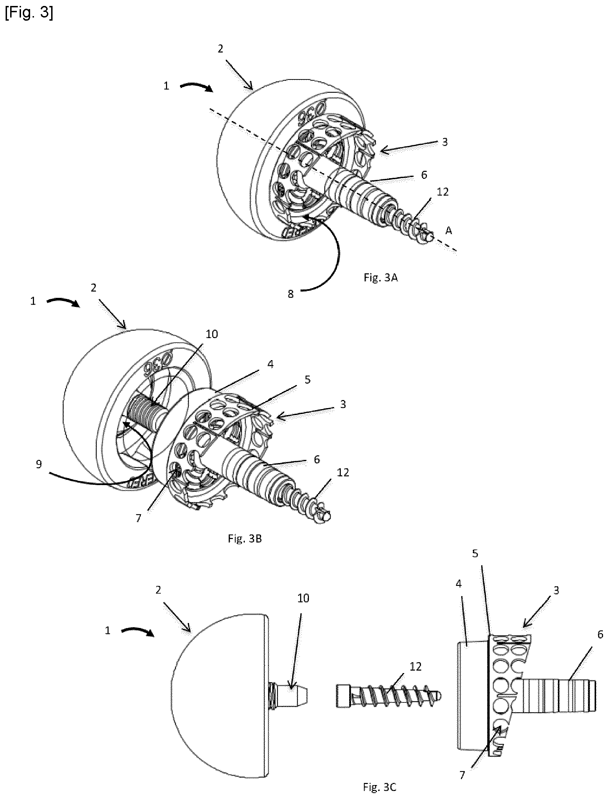

Afin de permettre une pénétration plus profonde au sein de la glène, et donc une meilleure fixation finale, le plot 6 comprend soit à son extrémité distale un élément rapporté 11 permettant d'allonger le plot central 6 (figures 1A à 2B), soit une vis 12 dont l'extrémité s'étend au-delà de l'extrémité distale du plot 6 permettant aussi d'allonger le plot central 6 (figure 3A à 4C). De telles variantes alternatives de réalisation peuvent être inversées de sorte que le premier mode de réalisation comprend une vis 12 et que le deuxième mode de réalisation soit pourvu de l'élément rapporté 11.

Dans les modes de réalisation et variantes représentées, la base 4, le plot 6 et le support de greffon 5 forment un ensemble monobloc et sont réalisés en alliage de titane, avantageusement revêtu de titane et/ou de HAP (hydrocarbure aromatique polycyclique). Bien entendu, tout autre matière ou alliage satisfaisant les requis propres aux métaglènes et à leur impaction, au sein des glènes des patients, peut être envisagé par l'homme du métier. Alternativement, au moins un de ces trois composants peut être fabriqué indépendamment des deux autres et assemblé à ceux-ci ultérieurement.

Dans le premier mode de réalisation (figure 1A à 2B), la métaglène 3 est latéralisée grâce à la présence du support de greffon 5 qui s'étend radialement de la base 4, d'une longueur L1 ou L2 constante. Celle-ci permet en outre de préserver l'intégrité du greffon (non représenté mais réparti sur l'ensemble de la surface interne 8 du support 4) lors de l'impaction de la métaglène 3 dans la glène du patient, ainsi que lors de l'impaction de la glénosphère 2 sur la métaglène 3. Ainsi, lorsque la glène du patient est usée principalement en son centre, il est possible, grâce à la métaglène 3 selon le premier mode de réalisation, de latéraliser le centre de rotation de l'articulation de sorte que celui-ci se retrouve à l'emplacement adéquat pour permettre de solliciter le muscle deltoïde dès les premiers degrés d'abduction, tout en permettant une bonne reconstitution du stock osseux entre le greffon et la glène via notamment les orifices traversants 7 du support de greffon 5.

Ces orifices traversants 7 peuvent être identiques ou avoir des formes différentes de l'un à l'autre. Ils sont avantageusement répartis sur la totalité de la surface interne 8 du support de greffon 5 de sorte que les liaisons osseuses entre la glène et le greffon puissent se faire de la manière la plus homogène possible.

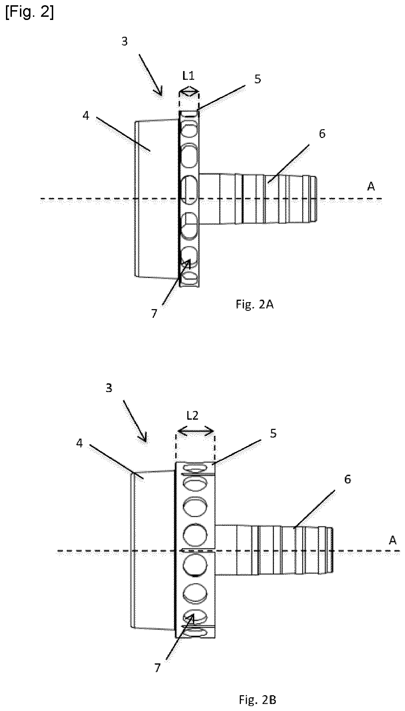

Avantageusement, les longueurs L1 et L2 sont comprises entre 2 et 12 mm (figures 2A et 2B), afin de respecter les standards en matière de latéralisation du centre de rotation de l'articulation.

Dans le deuxième mode de réalisation (figure 3A à 4C), la métaglène 3 est du type « wedge » grâce à la présence du support de greffon 5 qui s'étend radialement de la base 4 d'une longueur (L3, L4, L4', L5, L5') qui varie localement. Autrement dit, le support de greffon 5 est plus long sur l'un des ses côtés, formant ainsi un angle qui permet *in fine* de déporter le centre de rotation de l'articulation à l'emplacement souhaité, lorsque l'usure de la glène du patient est asymétrique.

Avantageusement, les variations de longueur forment un angle compris entre 5° et 20°, ce qui permet au chirurgien de s'adapter à tout type d'usure asymétrique de la glène.

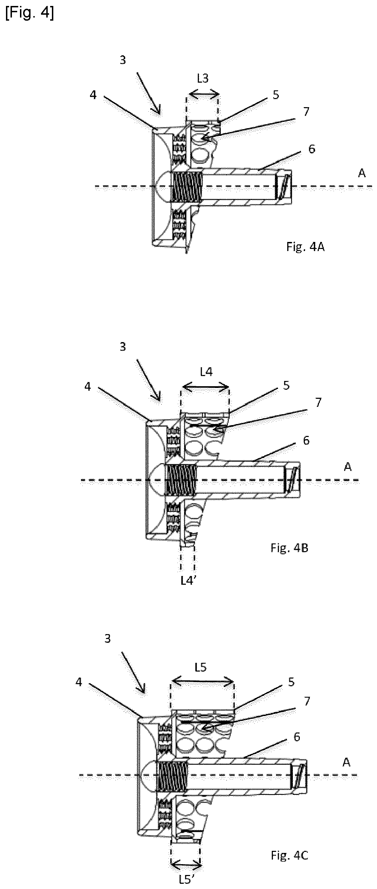

Comme représenté à la figure 4A, le support de greffon 5 s'étend uniquement sur une portion du pourtour de la base 4. Comme représenté aux figures 4B et 4B, le support de greffon 5 de la métaglène 3 peut combiner une fonction de latéralisation (la première portion du support de greffon 5 ayant une épaisseur constante) et une fonction de compensation d'une usure asymétrique de la glène (seconde portion pour laquelle le support 5 ne présente plus une épaisseur constante). La combinaison de ces deux paramètres permet de remettre le centre de rotation au bon endroit et dans le bon axe et de répondre ainsi aux cas d'usure de la glène les plus complexes.

On fixe un implant glénoïdien de prothèse d'épaule inversée comprenant une métaglène selon l'invention comme suit :
- on arase la glène ;
- on impacte la métaglène 3 dans la glène ;
- on met en place des vis de fixation ;
- on impacte et on visse une glénosphère 2 sur la métaglène 3.

L'invention n'est pas limitée aux modes de réalisation présentés et d'autres modes de réalisation apparaîtront clairement à l'homme du métier.

### Liste de références

1 : implant glénoïdien de prothèse d'épaule inversée
2 : glénosphère
3 : métaglène
4 : base
5 : support de greffon
6 : plot
7 : orifice traversant du support de greffon
8 : surface interne du support de greffon
9 : logement de la glénosphère
10 : vis de serrage
11 : élément rapporté
12 : vis
A : axe de révolution de la métaglène

## Revendications

1. Métaglène (3) d'un implant glénoïdien (1) de prothèse d'épaule inversée, destinée à être assemblée avec une glénosphère (2), la métaglène (3) comprenant :
- une base (4) de forme cylindrique et configurée pour être insérée au sein d'un logement (9) de la glénosphère (2),
- un plot (6) de forme cylindrique s'étendant depuis le centre de la base (4) selon un sens d'impaction de la métaglène (3) dans la glène d'un patient,
- un support de greffon (5) de forme cylindrique et s'étendant depuis le pourtour de la base (4) selon le sens d'impaction de la métaglène (3), le support de greffon (5) présentant au moins un orifice traversant (7),
la base (4), le plot (6) et le support de greffon (5) ayant un axe de révolution (A) commun.

2. Métaglène (3) selon la revendication précédente, dans laquelle le support de greffon (5) comprend une pluralité d'orifices traversants (7) répartie sur la surface interne (8) dudit support (5).

3. Métaglène (3) selon l'une des revendications précédentes, dans laquelle le support de greffon (5) s'étend radialement d'une longueur (L1, L2) constante.

4. Métaglène (3) selon la revendication précédente, dans laquelle la longueur (L1, L2) constante est comprise entre 2 et 12mm.

5. Métaglène (3) selon l'une des revendications 1 ou 2, dans laquelle le support de greffon (5) s'étend radialement d'une longueur (L3, L4, L4', L5, L5') qui varie localement.

6. Métaglène (3) selon la revendication précédente, dans laquelle la variation maximale entre deux extrémités du support de greffon (5) est comprise entre 2 et 20mm.

7. Métaglène (3) selon l'une quelconque des revendications précédentes, dans laquelle la base (4), le plot (6) et le support de greffon (5) sont monoblocs, de préférence réalisés en alliage de titane, de préférence encore réalisés en alliage de titane revêtu de titane et/ou de HAP.

8. Métaglène (3) selon l'une quelconque des revendications précédentes, dans laquelle le plot (6) comprend, à son extrémité distale, un élément rapporté (11) permettant d'allonger le plot (6).

9. Métaglène (3) selon l'une quelconque des revendications 1 à 7, dans laquelle le plot (6) comprend une vis (12) dont l'extrémité s'étend au-delà de l'extrémité distale du plot (6) permettant d'allonger le plot (6).
